# EUROPEAN PATENT SPECIFICATION

(11) **EP 4 259 056 B1**
(45) Date of publication and mention of the grant of the patent: **27.08.2025**
(21) Application number: 21848309.7
(22) Date of filing: 01.12.2021
(51) Int. Cl.: A61F 13/26

(54) **REUSABLE TAMPON INSERTING APPLICATOR**
WIEDERVERWENDBARE TAMPONAPPLIKATOR
APPLICATEUR DE TAMPON RÉUTILISABLE

(30) Priority: 09.12.2020 SI 202000230
(43) Date of publication of application: 18.10.2023
(73) Proprietor: TOSAMA Tovarna sanitetnega materiala d.o.o., 1230 Domzale (SI)
(72) Inventor: KOVAC, Gregor, 1251 Moravce (SI); SOLINC SIMNIC, Mojca, 1131 Ljubljana (SI); BRDNIK, Tomaz, 1293 Smarje Sap (SI)
(74) Representative: Borstar, Dusan
(86) International application number: PCT/SI2021/000010
(87) International publication number: WO 2022/124999

(56) References cited:
- CN-A- 111 888 090
- US-A1- 2017 020 744
- NATURISIMO: "DAME Reusable Tampon Applicator Set", 13 October 2020 (2020-10-13), XP055907771, Retrieved from the Internet <URL:https://www.youtube.com/watch?v=HcLT-okA9sY> [retrieved on 20220331]

## Description

The invention refers to an applicator, which is suitable for inserting of a tampon into a body cavity. Pursuant to the International Patent Classification such inventions in the field of common life necessities belong to medicine and hygiene, namely to tampons, in particular to menstrual hygienic tampons and accessories therefor, consequently to the class A 61 F 13/20, wherein the applicators as such, comprising a means for pushing a tampon as placed within an inserting tube, belong to the class A 61 F 13/32.

The invention is based on a problem of how to design an applicator for safe and hygienic insertion of each tampon into each body cavity, wherein such an applicator would have to be reusable due to environment protection related to the tendency of reducing the amount of disposed plastic waste, which means, that the user should get an opportunity to store it for the purpose of reusing it after the insertion of the tampon, as well as to clean it and prepare it for repeated use by commonly used cleaning means, while at the same time it would be desired, if the same applicator could be used for inserting of different tampons, which are at present commercially available and, which despite to having a similar shape differ from each other with regard to expected absorption capacity, namely in the amount of fibers comprised therein, which reflects in their weight and also the size.

An applicator for inserting a tampon into a body cavity is disclosed in EP 1 688 112 A1. Such an applicator comprises a substantially cylindrical, on the one terminal portion semi-spherically shaped tube, which is on its remaining terminal position thinned and into which a tampon is insertable prior to ejection thereof. A tampon, which is suitable for cooperating with such an applicator, is a so-called digital tampon, which is formed by radially compressing of a blank, which consists of a helically wound fibrous band and is on its front inserting terminal portion fitted with a slightly rounded head and on its rear terminal portion fitted with a flexible string, which enables removal of a moisturized tampon from a body cavity after the use. Said semi-spherical terminal portion is adjusted for inserting the applicator into a body cavity and is formed by a plurality of flexible flaps, which are uniformly distributed along the circumference of the tube and deflected radially inwards, but can be during ejection of the tampon also deflected outwardly apart from each other in order enable the tampon to exit from the inside of the tube. Said thinned rear terminal portion of said tube is relatively thin and serves as a tubular guide, through which the piston rod passes into the inside of the thicker piston area, which reaches into said tube and the purpose of which is pushing and ejecting the tampon from the applicator into each body cavity. In the case, where the piston rod is hollow, the retaining string, which the tampon is fitted with for the purpose of removal from each body cavity after use, may extend through said piston rod, which then enables reliable guiding thereof and prevents jamming of said string during the deployment of the tampon from the applicator. Such an applicator comprises a flexible thermoplastic material and could in this regard be reusable. However, due to pretty complex process of assembling and disassembling as required for inserting the tampon and also due to complicated and questionable cleaning after the use, it is suitable for single use only. Furthermore, in addition to said single use, such applicator is due to its extreme length, which is two or three times longer than as the length of the tampon itself, even in the case of just storing the tampon prior to the use in view of its dimensions significantly larger than several other applicators as known from the state of the art, which in the context of its convenience certainly presents a serious drawback, and additionally contributes to the volume of unnecessarily disposed biologically non-degradable and therefore environmentally unfriendly waste.

An applicator for storing and inserting of a tampon into a body cavity, which is some smaller in view of its overall volume, is disclosed in WO 2011/115586 A1, while an alternative version of such applicator was also published as WO 2006/099944 A1. Such an applicator comprises two coaxial tubes consisting of biologically non-degradable thermoplastic material, namely an external tube as well as an internal tube, which is tightly matching inserted within the external tube and is moveable to and fro along the external tube. The external tube is on its rear end portion completely open and allows insertion of the internal tube therein, and is in principle open also on the opposite inserting end portion, where however it is furnished with flexible flaps, which in their unloaded state form an approximately semi-spherical area, which enables insertion of the applicator into a body cavity. Also the internal tube is on its rear end portion completely opened and is suitable for inserting each tampon therein, while on its front end portion it is furnished with circumferentially distributed and in the direction towards the front end of the external tube protruding and at the same time slightly inwards deflected flaps. The corresponding tampon, which is suitable for cooperation with such an applicator in this case needs to be a so-called applicator tampon, which is similarly to the previously discussed digital tampon manufactured of radially compressed blank, obtained by a helically winding of a fibrous band, and which is moreover on its front inserting end portion equipped with a thickened and slightly rounded head, while it is on its rear end it is also fitted with a flexible string, which enables the removal of a moisturized tampon from a body cavity after the use. Before the use of the applicator, the internal tube is inserted inside of the external tube, while the tampon is inserted inside of the internal tube, although its thickened head protrudes outside of the internal tube and is engaged by retaining teeth on the internal surface of the external tube. During insertion of the tampon, when the external tube with its rounded end enters each body cavity, while retaining the tampon in its position, the tube is almost completely pulled from the external tube into the position, where the tampon exits from the internal tube and said inwards bent flaps of the internal tube are located behind the tampon. When the internal tube is pushed into the external tube, the internal tube also pushes a tampon located in front of it, so that the tampon passes among the spread flexible flaps of the external tube and is in such manner ejected from the inside of the applicator into a body cavity. Storing of a tampon in such an applicator is in view of hygienic requirements quite acceptable, and also insertion of the tampon as such can be carried out fast and in a suitable manner. However, after the use each user usually simply discards such an applicator, which in practice means that one such applicator is used per each used tampon, which leads to excessive and unnecessary generating of plastic waste, which is biologically practically non-degradable.

Those skilled in the art are aware that in view of the expected absorption capacity, namely of the amount of liquid, which the tampon is capable of absorb, tampons in different sizes are commercially available, which despite to their substantially similar shape differ from each other in the amount of absorbent fibers comprised therein, which is in the practice expressed by the weight of a tampon as well as by different diameters and lengths of a tampon. Regarding the previously discussed case it is pretty clear, that the applicator, in view of its dimensions, needs to be precisely adjusted to each desired size of the tampon, in particular to diameter and the length thereof, since otherwise by insertion of the tampon neither sliding of the tampon along the inside of the applicator tube together with a required retaining, nor ejection of the tampon from the external tube would be possible. This means that each manufacturer must take care about mass production of different applicators, which correspond to different types of tampons, which is then unavoidable expressed in higher final price of the product, which could however be significantly cheaper in the case of using a single universal applicator.

At the first glance one might conclude that such an applicator could generally also be reusable, but in practice on the basis of various researches and questionnaires it was shown, that is in the practice such approach would still not be automatically feasible in the mass consumption from average users. Namely, such an applicator generally cannot not be easily and efficiently cleaned immediately after the use and then also properly prepared for reuse. Namely, just loading of the tampon by first inserting it correctly into the internal tube and then inserting the internal tube together with the tampon in a correct manner into the external tube, establishing of properly retaining of the tampon inside of the tube, pulling the internal tube into the correct position and then ejecting the tampon from the internal tube into each body cavity is a process, which is in fact pretty complicated and is obviously too difficult for an average user. In addition to that, in order to reuse the applicator, the user would have to possess so much different types of applicators, which would be suitable for being loaded with different types of tampons, which are mostly or at least occasionally used, which in practice appears to be quite complicated and also pretty expensive.

Having come to terms with said findings, tampon manufactures started to search for different solutions in the field of reusable applicators. One of solutions in this direction, which is disclosed in WO 2017/015426 A1, comprises and external tube, into which an internal tube is be coaxially insertable in order to function as a piston suitable for pushing the tampon placed in front of it. The external tube is on the inserting end portion slightly rounded and is therefore suitable for insertion into a body cavity, and is moreover made of a suitably flexible material and furnished with a longitudinal gap, thanks to which it can be bent apart either for the purpose of loading a tampon therein or pushing the tampon from it, but in particular also for the purpose of easier cleaning. A cartridge is also foreseen, into which the applicator can be inserted immediately after the use, namely before cleaning and preparation for reuse together with an unused tampon. Due to required flexibility of the external tube material, a serious problem may arise in view of reliable guidance of the tampon in the correct direction during its ejection into a body cavity. A still further problem refers to hygienic aspects, since during insertion of the tampon into the external tube, as well as later during the removal of the applicator from said cartridge the user must touch the external surface of the external tube with fingers also in those areas, which during insertion of the applicator into a body cavity come into contact with the mucosa. Furthermore, it also does not seem to be quite impossible, that by expansion of the external tube inside of the body cavity during ejection of the tampon, which is then followed by retrieval of such expanded tube back into its initial state, a part of human tissue or mucosa may enter the area of the longitudinal gap, which might then remain pinched, which could cause an injury. A conceptually similar solution, which could lead to similar drawbacks, is also described in US 2011/0040234 A1.

A further solution for a reusable applicator is described in WO 2020/021137 A1. Also in this case, the solution again proposes an applicator with an external tube, into which on the one of its end portions a piston is coaxially inserted, while on the remaining end it is for the purpose of insertion into a body cavity slightly rounded and furnished with an aperture, so that on this end portion the tampon can be ejected from its inside into a body cavity. Said external tube and said piston are generally assembled in such a manner that the piston is reliably guided in the suitable direction by means of a guide, which is designed on the closed end portion of the external tube. In order to enable insertion of the tampon, the external tube is furnished with a relatively large longitudinal cutout in the size of a silhouette of the tampon along its entire length, while said cutout passes through the significant part of the length of the external tube. This enables insertion of the tampon into the tube through said cutout in the state, in which the piston is completely retracted. In this case it is also foreseen, that before the use the external tube together with the tampon loaded therein can be inserted into a cartridge, by which then the tampon is safely and hygienically stored prior to it use. Also in this case, at least during the movement of the piston and ejection of the tampon, the user must touch the external tube by fingers in those areas, which come into contact with the mucosa after insertion into a body cavity. When bearing in mind that the piston is inserted inside the external tube in a practically non-removable manner, each potential cleaning of this generally reusable applicator should be a challenge due to difficult accessible areas inside of the tube, in particular in the area of the guidance of the piston and also on the opposite end portion, where the quality of cleaning is questionable, which may by repeating or long-term use lead to a high risk of deposition of excrements and concentration of microorganisms. In addition, it is also quite possible, that during removal of the external tube from the cartridge, within which it is stored together with the tampon loaded therein, said cutout for inserting the tampon is facing at least approximately downwards, namely in the direction towards the ground. Since said cutout should enable an easy and simple inserting of the tampon into the tube, in said position the tampon may also easily fall out due to gravity. A tampon, which falls down on the ground anywhere, is then regularly not usable anymore. And this problem may then be followed by another one, namely that the user does not have a backup tampon at hand, or that she has a backup tampon, but she is unable to wash her hands prior to loading it into the applicator, which may also jeopardize sterility of the tampon and the applicator.

It would certainly make sense to avoid all aforementioned shortcomings of known applicators by an improved applicator, which would solve the previously explained technical problem.

Further relevant tampon applicators are known in the prior art and are disclosed in US 2017/020744 A1 and CN 111 888 090 A, or also retrieved from the internet via URL:https:/www.youtube.com/watch?v=HcLT-okA9sY (Naturisimo: "DAME Reusable Tampon Applicator SET".

The proposed invention generally refers to a reusable tampon inserting applicator, which is suitable for inserting of a hygienic tampon into a human body cavity,
wherein such applicator comprises
   - a substantially cylindrical inserting tube, which is on the one hand adapted for receiving and storage of the tampon, which is furnished with a retaining string for removal thereof from each body cavity, prior to insertion of said tampon into each body cavity, and on the other hand also for inserting the applicator as such into said body cavity,
   - a piston, which is placed within said inserting tube and is and controlled from the outside and displaceable to and fro along the inserting tube, wherein said piston is suitable for pushing the tampon along the inserting tube and also for ejecting the tampon from said inserting tube by simultaneously inserting it into a body cavity,
   - and moreover also a protective cartridge, into which at least the inserting tube of the applicator can be inserted for the purpose of keeping said inserting tube together with said tampon safely stored inside of said protective cartridge and protected from external influences,
wherein said inserting tube consists of a physiologically acceptable and flexible i.e. bendable thermoplastic material, which is suitable for repeated use, and which comprises an exit opening, which is located on the insertion end portion thereof and is surrounded with a plurality of slightly inwards deflected flaps, which are distributed uniformly around the circumference and which on the one hand in their initial position and unloaded state all together form a substantially semispherical composition, which allows the insertion tube and consequently also the applicator to be inserted into a body cavity, while on the other hand said flaps are thanks to their flexibility and bending capability also deflectable at least to such extent, that the tampon is allowed to exit from the inserting tube into a body cavity, while said inserting tube is on its remaining end portion fitted with an entry opening, which is suitable for loading the tampon into the inserting tube,
and wherein in the area of said entry opening a guide is foreseen in the inserting tube, so that said guide is adjusted for guiding of the piston in its longitudinal direction and during ejection of the tampon also in the longitudinal direction of said inserting tube,
and wherein said piston, which similarly like said inserting tube consists of a physiologically acceptable and sufficiently flexible i.e. bendable thermoplastic material, which is suitable for repeated use, comprises a piston rod, which is inserted throughout said guide of the inserting tube and is moveable to and fro along said guide, wherein said piston rod is on its towards the inserting tube facing terminal portion fitted with a thickened and properly shaped pushing section, adapted for resting on the surface of the tampon within the inserting tube, while on its remaining terminal portion, which is located outside of the inserting tube, the piston rod is equipped with a control section, which is ergonomically adjusted for being in contact with a human finger in order to enable controlling each movement of the piston along the insertion tube when pushing the tampon from the interior of the inserting tube into a body cavity, or also for just pushing the piston itself towards the interior of the inserting tube, wherein the length of said piston rod is determined in such a manner, that by fulfilling of all required ergonomic parameters for manipulating with the applicator, the piston can still be moved for at least such extent, which is sufficient for ejection of the tampon out from the inserting tube,
and wherein said protective cartridge, which similarly like the inserting tube and the piston consists of a physiologically acceptable and flexible i.e. bendable thermoplastic material, which is also suitable for repeated use, is designed as a hollow and from all sides enclosed housing, but is furnished with an inlet opening, through which tightly and protected from undesired removal at least such portion of the inserting tube of the applicator can be inserted, which should be during the use, namely just prior to ejection of the tampon as stored inside the inserting tube, inserted into a body cavity,
wherein the inserting tube is in the area around said entry opening on its rear end portion and at a sufficient distance apart from the exit opening on its inserting terminal portion, which exceeds the length of the tampon, furnished with a rigid flange, and also that in the area of said flange, the guide of the piston rod of the piston is surrounded by a suitably rigid and flexible housing, which is in a detachable manner arrested to said flange of the inserting tube in a position, in which the piston rod of the piston and the insertion tube are arranged coaxial with each other, namely in such a manner, that the guide is protected from undesired removal from the inserting tube either by pushing of the piston for the purpose of ejection of the tampon from the inserting tube upon insertion thereof into each body cavity, or during the removal of the inserting tube itself from the body cavity. and wherein said housing of the guide is attachable to the flange of the inserting tube and is arrested thereon by means of mutual cooperation between on the one hand a circumferentially extending groove, which is available on the away from the inserting terminal portion of the inserting tube facing external surface of the flange, and on the other hand a circumferentially extending rib, which is available on the internal surface of the housing of the guide of the piston, which is to be arrested into said groove.
and wherein said rib may alternatively also be arranged on the flange, while in such case said groove is arranged on the housing of the guide.

The invention however proposes that the flange of the inserting tube is pivotally attached to the housing of the guide, such that the guide, together with the piston rod of the piston, which passes through said guide, is moveable between its first terminal position, in which the inserting tube and the piston rod are coaxially aligned, into its second terminal position, in which the longitudinal geometric axes of the inserting tube and the piston rod extend parallel with each other, or into any other intermediate position between said terminal positions, when desired.

In a preferred embodiment of the invention said flange of the inserting tube and said housing of the guide of the piston rod of the piston are firmly and permanently interconnected with each other by means of a flexible bond, which presents an integral part both of the housing of the guide and simultaneously also of the flange of the inserting tube. A further embodiment may however also be foreseen, in which said flange of the inserting tube and said housing of the guide of the piston are connected with each other pivotally, but in a detachable manner.

The applicator according the invention further provides a possibility that said flange is furnished with a cutout, which is adjusted to allow passing of the retaining string there-through, when the tampon is loaded within the insertion tube, towards the exterior of said inserting tube of the applicator.

The invention further provides that in a position, in which the piston is totally pushed inwards into the inserting tube to a terminal position thereof, the piston rod is arrested and secured against any further uncontrolled movement in the longitudinal direction of the applicator. In the one of the embodiments, in such context and for the purpose of said protection against uncontrolled movement of the piston rod in the terminal of the piston, when it is fully pushed totally pushed inwards into the inserting tube, a circumferentially extending groove is arranged on the internal surface of the guide, and a circumferentially extending rib is arranged on the external surface of the piston rod in the adjacency of the control section, which can be arrested into said groove on the guide. However, another embodiment provides that due to protection of the piston rod against undesired movement thereof in said terminal position on the internal surface of the guide, a groove is foreseen on the piston rod, while on the guide a circumferentially extending rib is available, which is adjusted for cooperation with said groove.

Still further, the inserting tube is tightly insertable into the protective cartridge and is in its inserted state jammed and protected against undesired removal due to its conical external surface, or is tightly insertable into the protective cartridge and is in its inserted state jammed and protected against undesired removal by means of at least one circumferentially extending groove on the internal surface of the protective cartridge which is suitable for cooperation with at least one circumferentially extending rib on the external surface of the inserting tube.

At least one benefit of the invention may also result from the feature, that the insertion tube for receiving the tampon, and the piston for pushing the tampon through the ejecting opening of the insertion tube, are adjusted to cooperate with a tampon, which is either an applicator tampon or a digital tampon.

Now the invention will also be described in more details on the basis of embodiments and in connection with to the attached drawings, wherein
- Fig. 1: presents an embodiment of a reusable hygienic tampon inserting applicator in isometric view, which includes a corresponding protective cartridge, during the insertion of a tampon into a body cavity, which is not shown;
- Fig. 2: an applicator according to Fig. 1 with a tampon stored therein and together with a belonging protective cartridge, presented in an explosion view and in a state just before insertion of the tampon into a not-shown body cavity;
- Fig. 3: an applicator according to Figs. 1 or 2, which is suitable for being cleaned, and prior to introducing the tampon into the applicator;
- Fig. 4: an applicator according to Fig. 3 after insertion of the tampon into the applicator;
- Fig. 5: an applicator according to Figs. 1 - 4 in an assembled state, namely after being cleaned and ready for the forthcoming insertion of the tampon by reusing of said applicator, presented as a cross section along its diametric plane;
- Fig. 6: the detail A according to Fig. 5;
- Fig. 7: the detail B according to Fig. 5; and
- Fig. 8: again an applicator according to Fig. 3, namely in a state, in which it is suitable for being cleaned and prior to introduction of a subsequent tampon into the applicator, which here presented as a cross section along its diametric plane.

Reusable applicator 1 for inserting a hygienic tampon 8 into each body cavity, as shown in Figs. 1 and 2, generally comprises an essentially cylindrical inserting tube 2, which is suitable for receiving of said tampon 8 and inserting it into each body cavity, a piston 3, which suitable for ejecting said tampon 8 out of said tube 2 into a body cavity, as well as a protective cartridge 4, which is suitable for storing said inserting tube 2 together with said piston 3 prior to application of the applicator 1 as such, and also after that.

The inserting tube 2 is adapted on one hand for receiving and storing of said tampon 8, which is equipped with a retaining string 80 for removal of the tampon 8 from each body cavity, prior to inserting of said tampon 8 into a body cavity, and on the other hand also for inserting of the applicator 1 as such into a body cavity. On the other hand, said piston 3, which can be controlled from the outside and is moveable to and fro within inserting tube 2 and along thereof, is suitable for pushing said tampon 8 along said tube 2 and consequently for ejecting of the tampon 8 from said tube 2 into a body cavity. The applicator 1 additionally comprises a protective cartridge 4, into which the inserting tube 2 of the applicator 1 can be inserted for the purpose of maintaining said inserting tube 2 together with said tampon 8 safely stored inside of said protective cartridge 4 and herewith protected against the external influences. Prior to inserting of the tampon 8 into a body cavity and bringing it in contact with mucosa as present therein, it is necessary to assure that the tampon 8 is flawless with regard to hygienic aspects. On the other hand, after each use of the applicator 1, namely after insertion of the tampon 8 into a body cavity and removal of the tube 2 from a body cavity after that, at least the inserting tube 2 is usually covered by body excrements, which means that in such state its reuse would be evidently inappropriate, and is even problematic for being deposited or stored anywhere, and this is the reason why the protective cartridge 4 is foreseen, which is capable for storing the applicator 1 after the use and prior to cleaning thereof.

The inserting tube 2 as such consists of a physiologically acceptable, and flexible i.e. suitably bendable thermoplastic material, which is suitable for cleaning and repeated use and which is on its inserting end portion 21 furnished with an exit opening 210, which is surrounded with a plurality of slightly inwardly bended flaps 211, which are equidistantly distributed around the circumference thereof, and which on one hand in their initial position and unloaded state all together form a substantially hemispheric composition, which allows to the inserting tube 2 and consequently also to the applicator 1 as such, insertion into a body cavity, wherein on the other hand said flaps 211 are thanks to their flexibility and bending capability deflectable at least to such extent, that ejecting of the tampon 8 from the inserting tube 2 into a body cavity is enabled, while on the other hand, said inserting tube 2 is on its remaining end portion 22 fitted with an entry opening 220, which is suitable for introduction of the tampon 8 into the inserting tube 2.

Furthermore, a guide 25 is foreseen in the inserting tube 2 in the area of said entry opening 220, and said guide 25 is adjusted for longitudinally guiding of said piston 3 in order to push out the tampon 8 in the longitudinal direction of said inserting tube 2.

Similarly like the inserting tube 2, said piston 3 also consists of a physiologically acceptable, and flexible i.e. suitably bendable thermoplastic material, which is suitable for cleaning and repeating use. Said piston 3 comprises a piston rod 30, which is inserted within the guide 25 of the inserting tube 2 and is to moveable to and fro along said guide 25, wherein said piston rod 30 is on its end portion 301, which is faced towards the inserting tube 2, fitted with a suitably thickened and properly shaped pushing section 31, which is adjusted to be rest on a corresponding surface of the tampon 8, when the last is placed within the inserting tube 2. Furthermore, the piston rod 30 is on its remaining end portion 32, which is all the time located outside the inserting tube 2, furnished with a control section 33, which is ergonomically shaped for the purpose of cooperation with a human finger, which enables suitable control of displacing the piston 3, in particular for the purpose of pushing the tampon 8 from the interior of the inserting tube 2 into a body cavity (Fig. 1), or also by pushing the piston 3 itself deeply into the inserting tube 2 due to storing the applicator 1 after the use (Fig. 5).

The length of said piston rod 30 is determined in such a manner, that by fulfilling of all required ergonomic parameters in view of handling the applicator 1, the piston 3 can still be moved at least to such extent, which should be sufficient for ejection of each particular tampon 8 of each predetermined length out from the interior of the inserting tube 2.

Similarly like the inserting tube 2 and the piston 3, also the protective cartridge 4 consists of a physiologically acceptable and flexible i.e. suitably bendable thermoplastic material, which is suitable for cleaning and repeated use. The protective cartridge 4 is created as a hollow and from all sides closed housing, which is however furnished with an entry opening 40, through which in a tightly fitting manner and protected from an undesired removal, at least that portion of the inserting tube 2 of the applicator 1 can be inserted, which is during the use, namely just prior to ejection of the tampon 8, which is prior to that stored within the inserting tube 2, inserted into a body cavity.

The inserting tube 2 is furnished with a suitably rigid flange 23, which is located in the area around said entry opening 220 on the belonging end portion 22 and at a sufficient distance from the exit opening 210 on the inserting end portion 21, which exceeds the length of the tampon 8. Furthermore, in the area of said flange 23, said guide 25 of the piston rod 30 of the piston 3 is surrounded by a suitably stable and flexible housing 250, which is by snapping and in a removable manner attachable to said flange 23 of the inserting tube 2 such that position the piston rod 30 of the piston 3 and the inserting tube 2 are coaxial. Upon attachment of the housing 250 of the guide 25 to said flange 23 of the inserting tube 2, the guide 25 is protected against undesired removal from the inserting tube 2 either by pushing the piston 3 for the purpose of ejecting the tampon 8 from the inserting tube 2 when inserting it into a body cavity, or also by retracting said inserting tube 2 from said body cavity.

In the shown embodiment of the applicator 1 according to the invention (Figs. 5 and 7), said housing 250 of the guide 25 is attachable to the flange 23 of the inserting tube 2 by snapping, which is on achieved thanks to mutual cooperation between, on the one hand, a circumferentially positioned groove 231, which is available on the external surface 230 of the flange 23, which is faced away from the inserting end portion 21 of the inserting tube 2, and, on the other hand, a circumferentially positioned rib 252, which is available on the internal surface 251 of the housing 250 of the guide 25 of the piston 3, which can be arrested by means of said groove 231.

In general, another embodiment of the applicator 1 is also possible, which is not shown in the drawings and in which said rib 253 is available on the flange 23, whilst said groove 231 is available on the housing 250 of the guide 25. Those skilled in the art should no doubt understand, that such interconnection between the housing 250 and the flange 23 could also be realized in several other ways and by using different arresting means, which might also be suitable for establishing a detachable interconnection between two coaxial elements by means of snapping, so that the previously described combination of the groove 231 and the rib 253 should not be interpreted as an essential limitation of the scope of the invention.

Said flange 23 of the inserting tube 2 is pivotally connected to the housing 250 of the guide 25 in such manner, that the guide 25 together with the piston rod 30 of the piston 3, which passes through said guide 25, is moveable from its first terminal position (Figs. 1, 2 and 5), in which the inserting tube 2 and the piston rod 30 are coaxially aligned, into its second terminal position (Figs. 3, 4 and 8), in which the longitudinal geometric axes of the inserting tube 2 and piston rod 30 extend parallel to each other, or optionally also into each desired intermediate position between said terminal positions.

In the shown embodiment of the applicator 1 according to the invention (Figs. 3, 4 and 8), said flange 23 of the inserting tube 2 and said housing 250 of the guide 25 of the piston rod 30 of the piston 3 are inseparably interconnected by means of a flexible bond 255, which presents an integral part of the housing 250 of the guide 25 and at the same time also of the flange 23 of the inserting tube 2.

An further embodiment is also possible, which is not shown on the drawings and in accordance to which said flange 23 of the inserting tube 2 and said housing 250 of the guide 25 of the piston 3 are pivotally interconnected, but in a detachable manner.

In the applicator 1 it is furthermore foreseen, that the flange 23 is furnished with a cutout 235, which is adapted to allow the retaining string 80 of the tampon 8, when being loaded within the inserting tube 2, to pass there-through towards the external area of said inserting tube 2 of the applicator 1. Consequently, upon inserting the tube 2 into a body cavity and during ejection of the tampon 8 by means of the piston 3, the retaining string 80 is all the time located behind the tampon 8, so that at least a portion of the string 80 is retained outside of the body cavity, which ensures that upon retraction of the applicator 1 and after the application of the tampon 8 as such, the tampon 8 can reliably be removed from the body cavity by means of said retaining string 80.

Furthermore, it is also foreseen, that the piston rod 30, together with the belonging piston 3, is in its terminal position (Fig. 5), in which the piston 3 is fully pushed into the inserting tube 2, by means of snapping protected against any further uncontrolled movement in the longitudinal direction of the applicator 1. This is in particular important, when the applicator 1 is cleaned and ready for use, or also after the use, when the inserting tube 2 is safely stored within the protective cartridge 4 and the piston 3 is pushed into said tube 2. Such approach on one hand allows achieving of the smallest possible volume of the applicator 1, which is required for storage thereof it is not in the use, and on the other hand also prevents undesired removal of the piston rod 30 including subsequent potential intrusion of undesired impurities into the inserting tube 2 via the piston rod 30. In the shown embodiment (Figs. 5 and 6), for the purpose of protection against uncontrolled movement of the piston rod 30 in its aforementioned terminal position, in which the piston 3 is fully pushed into the inserting tube 2, on the one hand a circumferentially extending groove 258 is foreseen on the internal surface 257 of the guide 25, while on the other hand a circumferentially extending rib 311, which can be snapped into said groove 258 on the guide 25, is available on the external surface 310 of the piston rod 30 adjacent to the control section 33.

A still further embodiment of the applicator 1 is also possible, which is not shown on the drawings and in which for the purpose of protection against uncontrolled movement of the piston rod 30 in its terminal position on the internal surface 257 of the guide 25, a groove 258 is foreseen on the piston rod 30, while on the guide 25 there is a circumferentially extending rib 311, which is suitable for cooperation with said groove 258.

The invention further proposes that the inserting tube 2 can be inserted into the protective cartridge 4 in a tightly fitting manner and is in its inserted state protected against undesired removal. In one of the embodiments of the invention, the inserting tube 2 can be wedged within the protective cartridge 4 thanks to its slightly conical external surface 200. In a preferred embodiment, the inserting tube 2 can be inserted into the protective cartridge 4 in a tightly fitting manner and is in its inserted state then also protected against undesired removal thanks snapping thereof due to cooperation between at least one circumferentially extending groove 401 on the internal surface 400 of the protective cartridge 4 and at least one circumferentially extending rib 201 on the external surface 200 of the inserting tube 2. Such embodiment is generally preferred, especially because a required matching between the inserting tube 2 and the protective cartridge 4 can be more loose, which means that even when the external surface 200 of the tube 2 is polluted after the use, there is no sticking, which could generally occur after the use and subsequently removing of such polluted tube 2 from the protective cartridge 4 prior to cleaning of the applicator 1 and preparation for reusing thereof. It is also foreseen according to the invention, that the flange 23 is on its external surface designed in such manner, that during the use of the applicator 1, namely when the control section 33 of the piston 3 is for the purpose of ejecting the tampon 8 from the inserting tube 2 pressed by one finger on a hand, said flange 23 actually establishes an ergonomically suitable support for at least one of the remaining fingers on the same hand of the user.

As evident from e.g. Fig. 5, in a preferred embodiment of the applicator 1 according to the invention, the housing 250 of the guide 25 is designed to be axially symmetric and in the shape of a cup, namely as a part of a hemisphere, and is in its equatorial ring area 253 interconnectable with said flange 23 of the inserting tube 2 and in its vertex area furnished with a flat surface 254, which is suitable for resting of the control section 33 of the piston 3 there-on in such a manner, that the housing 250 of the guide 25 and the control section 33 of the piston 3, together with the smooth surface of the protective cartridge 4 and the area of the flange 23 of the inserting tube 2, which is facing towards the exit opening 210, all together form an ergonomically and aesthetically acceptable external surface of the applicator 1. At the same time, the inserting tube 2 for receiving the tampon 8 and the piston 3 for pushing of the tampon 8 through the exit opening 210 of the inserting tube 2, are adjusted for cooperation with the tampon 8, which can be either an applicator tampon or a digital tampon.

When the inserting tube 2 and the housing with the guide 25 are uniformly designed and simultaneously manufactured, and are at the same time also pivotally interconnected by means of a flexible bond 255, then in a position according to Figs. 3, 4 or 8, a pushing section 31 of the piston 3, which is suitably thickened relatively to the piston rod 30 itself, can be pushed through the guide 25 by using a suitable force, wherein said pushing section 31 is thanks to its flexibility by moving through said guide 25 elastically deformed. As soon as the pushing section 31 has passed through the guide 25, the piston rod 30 is located within said guide 25, and since said piston rod 30 is thinner than the pushing section 31, it is then and is freely and easily moveable to and fro along said guide 25. Upon that, the guide 25 can be together with the housing 250 and the piston 3, which is inserted within the housing 250, swiveled into a coaxial position according to Fig. 1, and the piston 3 can be pushed into the tube 2, wherein said piston 3 is in its terminal position snapped in place to such an extent, that it is protected against undesired movement, e.g. a movement initiated by gravity or inertia due to its own weight, or the like. Upon that, the inserting tube 2 is introduced into the protective cartridge 4 together with the guide 25 and the belonging housing 250, as well as with the piston 3, which is inserted within said tube 2, which means the applicator 1 is then complete, and is as such suitable for commercialization and use (Fig. 8).

Prior to each use, the user first of all needs to pull the piston 3 out of the tube 2 and to swivel the guide 25, together with the housing 250 and the piston 3, aside into a position according to Fig. 3, upon which the user is able to load a desired tampon 8 through the entry opening 220 of the inserting tube 2 (Fig. 4). If the applicator 1 is in view of its dimensions suitable for inserting of the largest of the tampon 8, then such applicator 1 is also suitable for inserting of smaller types of the tampon 8 into a body cavity, regardless to that, if the tampon 8 is digital tampon or applicator tampon 8. After loading of the tampon 8, the guide 25 is positioned on the tube 2 together with the housing 250 and piston 3, namely in a position according to Figs. 1 or 2, wherein the tube 2 and piston rod 30 extend coaxially with regard to each other. After that, and just prior to ejection of the tampon 8, the applicator 1 including the area of the inserting tube 2 needs to be removed from the protective cartridge 4, which means that the external surface 200 of the inserting tube 2 has been until that moment kept despite to handling with the applicator 1 kept untouched, and is consequently still perfectly clean. Upon that, the inserting tube 2 can be introduced into a body cavity, while by pressing the control section 33 of the piston 3 by means of a finger, by resting at least one further finger of the same hand on the flange 23, the tampon 8 can be ejected into a body cavity through the exit opening 210 of the inserting tube 2 (Fig. 1). As soon as said ejecting of the tampon 8 is finished, the user can remove the applicator 1 from the body cavity and may either immediately clean and dry in order to prepare it for reuse, or may alternatively also introduce it back into the protective cartridge 4 in the area of the inserting tube 2, and push the piston3 inside the inserting tube 2 after that, just in order to clean the applicator 1 later-on, when it is practically possible with regard to each particular situation.

The applicator 1, which is created in such a way, is therefore without any doubt reusable, and is moreover also suitable for safe and hygienic insertion of tampons 8 into a body cavity. Besides, it is also obvious that a single applicator 1 can be used for inserting of various tampons 8, which are at present commercially available, and which may, despite to their similar shape, due to each expected absorption capacity, differ from each other with regard to content of fibers and consequently in their weight and size, in particular in diameter and length thereof.

## Claims

1. Reusable tampon inserting applicator (1), which is suitable for inserting of a hygienic tampon (8) into a human body cavity and comprises a substantially cylindrical inserting tube (2), which is on the one hand adapted for receiving and storage of the tampon (8), which is furnished with a retaining string (80) for removal thereof from each body cavity, prior to insertion of said tampon (8) into each body cavity, and on the other hand also for inserting the applicator (1) as such into said body cavity, as well as a piston (3), which is placed within said inserting tube (2) and is and controlled from the outside and displaceable to and fro along the inserting tube (2), wherein said piston (3) is suitable for pushing the tampon (8) along the inserting tube (2) and also for ejecting the tampon (8) out of said inserting tube (2) by simultaneously inserting it into a body cavity, and moreover also a protective cartridge (4), into which at least the inserting tube (2) of the applicator (1) can be inserted for the purpose of keeping said inserting tube (2) along with said tampon (8) safely stored inside of said protective cartridge (4) and protected from external influences,
wherein said inserting tube (2) consists of a physiologically acceptable and flexible i.e. bendable thermoplastic material, which is suitable for repeated use and which comprises an exit opening (210), which is located on its insertion end portion (21) and is surrounded with a plurality of slightly inwards deflected flaps (211), which are equidistantly distributed around the circumference and which, on the one hand, in their initial position and unloaded state all together form a substantially semispherical composition, which allows the insertion tube (2) and consequently also the applicator (1) to be inserted into a body cavity, while on the other hand said flaps (211) are thanks to their flexibility and bending capability also deflectable at least to such extent that the tampon (8) is allowed to exit from the inserting tube (2) into a body cavity, while said inserting tube (2) is on its remaining end portion (22) fitted with an entry opening (220), which is suitable for loading the tampon (8) into the inserting tube (2),
and wherein in the area of said entry opening (220) a guide (25) is foreseen in the inserting tube (2), so that said guide (25) is adjusted for guiding of the piston (3) in its longitudinal direction and during ejection of the tampon (8) also in the longitudinal direction of said inserting tube (2),
and wherein said piston (3), which similarly like said inserting tube (2) consists of a physiologically acceptable and sufficiently flexible i.e. bendable thermoplastic material, which is suitable for repeated use, comprises a piston rod (30), which is inserted throughout said guide (25) of the inserting tube (2) and is moveable to and fro along said guide (25), wherein said piston rod (30) is on its towards the inserting tube (2) facing terminal portion (301) fitted with a thickened and properly shaped pushing section (31), adapted for resting on the surface of the tampon (8) within the inserting tube (2), while on its remaining terminal portion (32), which is in located outside of the inserting tube (2), the piston rod (30) is equipped with a control section (33), which is ergonomically adjusted for being in contact with a human finger in order to enable controlling movement of the piston (3) along the insertion tube (2) when to pushing the tampon (8) out from the inserting tube (2) into a body cavity, or also for just pushing the piston (3) itself towards the interior of the inserting tube (2), wherein the length of said piston rod (30) is determined in such a manner, that by fulfilling all necessary ergonomic parameters for manipulating with the applicator (1), the piston (3) can still be moved for at least such extent, which it is sufficient for ejection of the tampon (8) out from the inserting tube (2),
and wherein said protective cartridge (4), which similarly like the inserting tube (2) and the piston (3) consists of a physiologically acceptable, flexible, and bendable thermoplastic material, which is also suitable for repeated use, is designed as a hollow and from all sides enclosed housing, but is furnished with an inlet opening (40), through which tightly and protected against undesired removal at least such portion of the inserting tube (2) of the applicator (1) can be inserted, which should during the use, namely just prior to ejection of the tampon (8) as stored inside the inserting tube (2), be inserted into a body cavity,
wherein the inserting tube (2) is in the area around said entry opening (22) on its rear end portion (22) and at a sufficient distance apart from the exit opening (210) on its inserting terminal portion (21), which exceeds the length of the tampon (8), furnished with a rigid flange (23),
and wherein in the area of said flange (23), the guide (25) of the piston rod (30) of the piston (3) is surrounded by a sufficiently rigid and flexible housing (250), which is in a detachable manner arrested to said flange (23) of the inserting tube (2) in a position, in which the piston rod (30) of the piston (3) and the insertion tube (2) are arranged coaxially with each other, namely in such a manner, that the guide (25) is protected from undesired removal from the inserting tube (2) either when pushing the piston (3) for the purpose of ejection of the tampon (8) from the inserting tube (2) upon insertion thereof into a body cavity, or also during removal of the inserting tube (2) itself from a body cavity,
and wherein said housing (250) of the guide (25) is attachable to the flange (23) of the inserting tube (2) and is arrested thereon by means of mutual cooperation between on the one hand a circumferentially extending groove (231), which is available on the away from the inserting terminal portion (21) of the inserting tube (2) facing external surface (230) of the flange (23), and on the other hand a circumferentially extending rib (252), which is available on the internal surface (251) of the housing (250) of the guide (25) of the piston (3), which is to be arrested into said groove (231),
and wherein said rib (252) may alternatively be arranged on the flange (23), while said groove (231) is arranged on the housing (250) of the guide (25),
**characterized in that**
the flange (23) of the inserting tube (2) is pivotally attached to the housing (250) of the guide (25), such that the guide (25), together with the piston rod (30) of the piston (3), which passes through said guide (25), is moveable between its first terminal position, in which the inserting tube (2) and the piston rod (30) are coaxially aligned, and its second terminal position, in which the longitudinal geometric axes of the inserting tube (2) and the piston rod (30) extend parallel with each other, or into any other intermediate position between said terminal positions, when desired.

2. Applicator according to Claim 1, **characterized in that** said flange (23) of the inserting tube (2) and said housing (250) of the guide (25) of the piston rod (30) of the piston (3) are firmly and permanently interconnected with each other by means of a flexible bond (255), which presents an integral part both of the housing (250) of the guide (25) and simultaneously also of the flange (23) of the inserting tube (2).

3. Applicator according to Claim 1, **characterized in that** said flange (23) of the inserting tube (2) and said housing (250) of the guide (25) of the piston (3) are pivotally, but in a detachable manner connected with each other.

4. Applicator according to any of the Claims 1 - 3, **characterized in that** the flange (23) is furnished with a cutout (235), which is adjusted to allow passing of the retaining string (80) there-through, when the tampon (8) is loaded within the insertion tube (2), towards the exterior of said inserting tube (2) of the applicator (1).

5. Applicator according to any of the Claims 1 - 4, **characterized in that** in a position, in which the piston (3) is totally pushed inwards into the inserting tube (2) to a terminal position thereof, the piston rod (30) is arrested and secured against any further uncontrolled movement in the longitudinal direction of the applicator (1).

6. Applicator according to Claim 5, **characterized in that** for the purpose of said protection against uncontrolled movement of the piston rod (30) in the terminal of the piston (3), when it is fully pushed totally pushed inwards into the inserting tube (2), a circumferentially extending groove (258) is arranged on the internal surface (257) of the guide (25), and a circumferentially extending rib (311) is arranged on the external surface (310) of the piston rod (30) in the direct vicinity of the control section (33), which can be arrested into said groove (258) on the guide (25).

7. Applicator according to Claim 5, **characterized in that,** due to protection the piston rod (30) against undesired movement thereof in said terminal position on the internal surface (257) of the guide (25), a groove (258) is foreseen on the piston rod (30), while on the guide (25) a circumferentially extending rib (311) is available, which is adjusted for cooperation with said groove (258).

8. Applicator according to any one of Claims 1 - 7, **characterized in that** the inserting tube (2) is tightly insertable into the protective cartridge (4) and is in its inserted state jammed and protected against undesired removal due to its conical external surface (200).

9. Applicator according to any one of Claims 1 - 7, **characterized in that** the inserting tube (2) is tightly insertable into the protective cartridge (4) and is in its inserted state jammed and protected against undesired removal by means of at least one circumferentially extending groove (401) on the internal surface (400) of the protective cartridge (4) which is suitable for cooperation with at least one circumferentially extending rib (201) on the external surface (200) of the inserting tube (2).

10. Applicator according to any one of Claims 1 - **9, characterized in that,** the insertion tube (2) for receiving the tampon (8), and the piston (3) for pushing the tampon (8) through the ejecting opening (210) of the insertion tube (2), are adjusted to cooperate with a tampon (8), which is either an applicator tampon or a digital tampon.

## Patentansprüche

1. Wiederverwendbarer Tamponeinführapplikator (1), der dafür geeignet ist, einen hygienischen Tampon (8) in einen Körperhohlraum eines Menschen einzuführen, und der ein im Wesentlichen zylindrisches Einführröhrchen (2) umfasst, das einerseits dafür geeignet ist, den Tampon (8), der mit einem Haltebändchen (80) zum Entfernen von diesem aus jedem Körperhohlraum versehen ist, aufzunehmen und aufzubewahren, bevor der Tampon (8) in jeden Körperhohlraum eingeführt wird, und andererseits auch dafür geeignet ist, den Applikator (1) als solchen in den Körperhohlraum einzuführen, sowie einen Kolben (3) umfasst, der innerhalb des Einführröhrchens (2) platziert ist und von außen gesteuert wird und entlang des Einführröhrchens (2) hin und her verschiebbar ist, wobei der Kolben (3) dafür geeignet ist, den Tampon (8) entlang des Einführröhrchens (2) zu schieben und den Tampon (8) auch aus dem Einführröhrchen (2) auszubringen, indem er gleichzeitig in einen Körperhohlraum eingeführt wird, und außerdem auch eine Schutzhülle (4) umfasst, in die zumindest das Einführröhrchen (2) des Applikators (1) eingeführt werden kann, um das Einführröhrchen (2) zusammen mit dem Tampon (8) im Inneren der Schutzhülle (4) sicher aufbewahrt und vor äußeren Einflüssen geschützt zu halten,
wobei das Einführröhrchen (2) aus einem physiologisch verträglichen und flexiblen, d. h. biegbaren, thermoplastischen Material besteht, das zur wiederholten Verwendung geeignet ist und das eine Ausgangsöffnung (210) umfasst, die sich auf dessen Einführendabschnitt (21) befindet und von einer Vielzahl von leicht nach innen abgelenkten Laschen (211) umgeben ist, die in gleichen Abständen um den Umfang verteilt sind, und die einerseits in ihrer Anfangsposition und einem nicht geladenen Zustand alle zusammen einen im Wesentlichen halbkugelförmigen Verbund bilden, durch den das Einführröhrchen (2) und folglich auch der Applikator (1) in einen Körperhohlraum eingeführt werden können, während die Laschen (211) andererseits dank ihrer Flexibilität und Biegefähigkeit auch zumindest bis zu einem solchen Ausmaß ablenkbar sind, dass der Tampon (8) aus dem Einführröhrchen (2) in einen Körperhohlraum austreten kann, während das Einführröhrchen (2) an seinem übrigen Endabschnitt (22) mit einer Eingangsöffnung (220) versehen ist, die dafür geeignet ist, den Tampon (8) in das Einführröhrchen (2) zu laden,
und wobei in dem Bereich der Eingangsöffnung (220) eine Führung (25) in dem Einführröhrchen (2) vorgesehen ist, so dass die Führung (25) zum Führen des Kolbens (3) in seiner Längsrichtung und während des Ausbringens des Tampons (8) auch in der Längsrichtung des Einführröhrchens (2) eingestellt wird,
und wobei der Kolben (3), der in ähnlicher Weise wie das Einführröhrchen (2) aus einem physiologisch verträglichen und ausreichend flexiblen, d. h. biegbaren, thermoplastischen Material, das für eine wiederholte Verwendung geeignet ist, besteht, eine Kolbenstange (30) umfasst, die durch die gesamte Führung (25) des Einführröhrchens (2) eingeführt und entlang der Führung (25) hin und her bewegbar ist, wobei die Kolbenstange (30) an ihrem in Richtung zum Einführröhrchen (2) weisenden Abschlussabschnitt (301) mit einem verdickten und passend geformten Schubabschnitt (31) versehen ist, der dafür geeignet ist, auf der Oberfläche des Tampons (8) innerhalb des Einführröhrchens (2) aufzuliegen, während die Kolbenstange (30) an ihrem übrigen Abschlussabschnitt (32), der sich außerhalb des Einführröhrchens (2) befindet, mit einem Steuerabschnitt (33) ausgestattet ist, der ergonomisch eingestellt ist, um mit einem menschlichen Finger in Kontakt zu stehen, um eine steuernde Bewegung des Kolbens (3) entlang des Einführröhrchens (2) zu ermöglichen, wenn der Tampon (8) aus dem Einführröhrchen (2) in einen Körperhohlraum geschoben werden soll, oder auch nur zum Schieben des Kolbens (3) selbst in Richtung zum Inneren des Einführröhrchens (2), wobei die Länge der Kolbenstange (30) derart bestimmt wird, dass durch Erfüllen aller notwendigen ergonomischen Parameter zum Handhaben des Applikators (1) der Kolben (3) immer noch zumindest bis zu einem solchen Ausmaß bewegt werden kann, das zum Ausbringen des Tampons (8) aus dem Einführröhrchen (2) ausreichend ist,
und wobei die Schutzhülle (4), die in ähnlicher Weise wie das Einführröhrchen (2) und der Kolben (3) aus einem physiologisch verträglichen, ausreichend flexiblen und biegbaren thermoplastischen Material besteht, das für eine wiederholte Verwendung geeignet ist, als hohles und von allen Seiten umschlossenes Gehäuse gestaltet ist, aber mit einer Einlassöffnung (40) ausgestattet ist, durch die eng und vor einem unerwünschten Entfernen geschützt zumindest ein solcher Teil des Einführröhrchens (2) des Applikators (1) eingeführt werden kann, der während der Verwendung, nämlich kurz vor dem Ausbringen des Tampons (8), der innerhalb des Einführröhrchens (2) aufbewahrt ist, in einen Körperhohlraum eingeführt werden sollte,
wobei das Einführröhrchen (2) im Bereich um die Eingangsöffnung (22) an seinem hinteren Endabschnitt (22) und in einem ausreichenden Abstand entfernt von der Ausgangsöffnung (210) an seinem Einführabschlussabschnitt (21), der die Länge des Tampons (8) übersteigt, mit einem starren Flansch (23) ausgestattet ist,
und wobei im Bereich des Flansches (23) die Führung (25) der Kolbenstange (30) des Kolbens (3) von einem ausreichend starren und flexiblen Gehäuse (250) umgeben ist, das auf lösbare Weise an dem Flansch (23) des Einführröhrchens (2) in einer Position arretiert ist, in der die Kolbenstange (30) des Kolbens (3) und das Einführröhrchen (2) koaxial ineinander angeordnet sind, und zwar auf eine solche Weise, dass die Führung (25) vor einem unerwünschten Entfernen aus dem Einführröhrchen (2), entweder beim Schieben des Kolbens (3) für den Zweck des Ausbringens des Tampons (8) aus dem Einführröhrchen (2) bei Einführen von diesem in einen Körperhohlraum, oder auch während des Entfernens des Einführröhrchens (2) selbst aus einem Körperhohlraum, geschützt ist,
und wobei das Gehäuse (250) der Führung (25) an dem Flansch (23) des Einführröhrchens (2) anbringbar ist und auf diesem durch gegenseitiges Zusammenwirken zwischen einerseits einer sich um den Umfang erstreckenden Nut (231), die auf der Außenfläche (230) des Flansches (23) vorhanden ist, die von dem Einführabschlussabschnitt (21) des Einführröhrchens (2) weg weist, und andererseits einer sich um den Umfang erstreckenden Rippe (252), die auf der Innenfläche (251) des Gehäuses (250) der Führung (25) des Kolbens (3) vorhanden ist, die in der Nut (231) zu arretieren ist, arretiert wird,
und wobei die Rippe (252) alternativ auf dem Flansch (23) angeordnet sein kann, während die Nut (231) auf dem Gehäuse (250) der Führung (25) angeordnet ist, **dadurch gekennzeichnet, dass**
der Flansch (23) des Einführröhrchens (2) schwenkbar an dem Gehäuse (250) der Führung (25) angebracht ist, so dass die Führung (25) zusammen mit der Kolbenstange (30) des Kolbens (3), die durch die Führung (25) hindurch geht, zwischen ihrer ersten Abschlussposition, in der das Einführröhrchen (2) und die Kolbenstange (30) koaxial ausgerichtet sind, und ihrer zweiten Abschlussposition, in der sich die geometrischen Längsachsen des Einführröhrchens (2) und die Kolbenstange (30) parallel zueinander erstrecken, oder in eine beliebige andere Zwischenposition zwischen den beiden Abschlusspositionen bei Bedarf beweglich ist.

2. Applikator nach Anspruch 1, **dadurch gekennzeichnet, dass** der Flansch (23) des Einführröhrchens (2) und das Gehäuse (250) der Führung (25) der Kolbenstange (30) des Kolbens (3) fest und dauerhaft mittels einer flexiblen Bindung (255) miteinander verbunden sind, die einen integralen Teil sowohl des Gehäuses (250) der Führung (25) als auch gleichzeitig des Flansches (23) des Einführröhrchens (2) darstellt.

3. Applikator nach Anspruch 1, **dadurch gekennzeichnet, dass** der Flansch (23) des Einführröhrchens (2) und des Gehäuses (250) der Führung (25) des Kolbens (3) schwenkbar, jedoch in einer lösbaren Weise miteinander verbunden sind.

4. Applikator nach einem der Ansprüche 1 bis 3, **dadurch gekennzeichnet, dass** der Flansch (23) mit einem Ausschnitt (235) ausgestattet ist, der eingestellt ist, um das Hindurchgehen des Haltebändchens (80) durch diesen zur Außenseite des Einführröhrchens (2) des Applikators (1) zu ermöglichen, wenn der Tampon (8) innerhalb des Einführröhrchens (2) geladen ist.

5. Applikator nach einem der Ansprüche 1 bis 4, **dadurch gekennzeichnet, dass** in einer Position, in der der Kolben (3) vollkommen nach innen in das Einführröhrchen (2) in eine Abschlussposition von diesem geschoben ist, die Kolbenstange (30) gegen jede weitere ungesteuerte Bewegung in der Längsrichtung des Applikators (1) arretiert und gesichert ist.

6. Applikator nach Anspruch 5, **dadurch gekennzeichnet, dass für** den Zweck des Schutzes vor einer ungesteuerten Bewegung der Kolbenstange (30) in dem Abschluss des Kolbens (3), wenn er vollkommen nach innen in das Einführröhrchen (2) geschoben ist, eine sich um den Umfang erstreckende Nut (258) auf der Innenfläche (257) der Führung (25) angeordnet ist, und eine sich um den Umfang erstreckende Rippe (311) auf der Außenfläche (310) der Kolbenstange (30) in unmittelbarer Nähe des Steuerabschnitts (33) angeordnet ist, die in der Nut (258) auf der Führung (25) arretiert werden kann.

7. Applikator nach Anspruch 5, **dadurch gekennzeichnet, dass** auf Grund des Schutzes der Kolbenstange (30) vor einer unerwünschten Bewegung von dieser in der Abschlussposition auf der Innenfläche (257) der Führung (25) eine Nut (258) auf der Kolbenstange (30) vorgesehen ist, während auf der Führung (25) eine sich um den Umfang erstreckende Rippe (311) vorhanden ist, die zum Zusammenwirken mit der Nut (258) eingestellt ist.

8. Applikator nach einem der Ansprüche 1 bis 7, **dadurch gekennzeichnet, dass** das Einführröhrchen (2) eng in die Schutzhülle (4) einführbar ist und in ihrem eingeführten Zustand durch ihre konische Außenfläche (200) verklemmt und vor einem unerwünschten Entfernen geschützt ist.

9. Applikator nach einem der Ansprüche 1 bis 7, **dadurch gekennzeichnet, dass** das Einführröhrchen (2) eng in die Schutzhülle (4) einführbar ist und in ihrem eingeführten Zustand verklemmt und vor einem unerwünschten Entfernen mittels mindestens einer sich um den Umfang erstreckenden Nut (401) auf der Innenfläche (400) der Schutzhülle (4) geschützt ist, die zum Zusammenwirken mit mindestens einer sich um den Umfang erstreckenden Rippe (201) auf der Außenfläche (200) des Einführröhrchens (2) geeignet ist.

10. Applikator nach einem der Ansprüche 1 bis 9, **dadurch gekennzeichnet, dass** das Einführröhrchen (2) zum Aufnehmen des Tampons (8) und der Kolben (3) zum Schieben des Tampons (8) durch die Ausbringöffnung (210) des Einführröhrchens (2) dafür eingestellt sind, mit einem Tampon (8) zusammenzuwirken, der entweder ein Tampon mit Applikator oder ein Tampon ohne Applikator ist.

## Revendications

1. Applicateur d'insertion de tampon réutilisable (1), qui est apte à insérer un tampon hygiénique (8) dans une cavité corporelle humaine et comprend un tube d'insertion essentiellement cylindrique (2), qui est adapté d'une part pour recevoir et stocker le tampon (8), qui est pourvu d'une ficelle de retenue (80) pour le retrait de celui-ci de chaque cavité corporelle, avant insertion dudit tampon (8) dans chaque cavité corporelle, et d'autre part également pour l'insertion de l'applicateur (1) lui-même dans ladite cavité corporelle, ainsi qu'un piston (3), qui est placé à l'intérieur dudit tube d'insertion (2) et est commandé depuis l'extérieur et déplaçable d'avant en arrière le long dudit tube d'insertion (2), dans lequel ledit piston (3) est apte à pousser le tampon (8) le long du tube d'insertion (2) et également à éjecter le tampon (8) hors dudit tube d'insertion (2) en l'insérant simultanément dans une cavité corporelle, et de plus également une cartouche de protection (4) dans laquelle au moins le tube d'insertion (2) de l'applicateur (1) peut être inséré afin d'assurer le stockage sécurisé dudit tube d'insertion (2) conjointement avec le tampon (8) à l'intérieur de ladite cartouche de protection (4), et de les protéger contre les influences extérieures,
dans lequel ledit tube d'insertion (2) est constitué d'un matériau thermoplastique pliable, c'est-à-dire flexible, physiologiquement acceptable, qui est adapté à une utilisation répétée et qui comprend une ouverture de sortie (210) qui est située sur sa partie d'extrémité d'insertion (21) et qui est entourée d'une pluralité de rabats (211) légèrement repliés vers l'intérieur, qui sont réparties de manière équidistante autour de la circonférence, et qui, d'une part, dans leur position initiale et à l'état non sollicité, forment ensemble une structure essentiellement hémisphérique qui permet l'insertion du tube d'insertion (2) et par conséquent également de l'applicateur (1) dans une cavité corporelle, tandis que d'autre part, lesdits rabats (211) sont également déformables du fait de leur souplesse et de leur capacité de flexion, au moins dans une mesure permettant au tampon (8) de sortir du tube d'insertion (2) dans une cavité corporelle, tandis que ledit tube d'insertion (2) est, sur sa partie d'extrémité restante (22), muni d'une ouverture d'entrée (220) qui est apte à permettre le chargement du tampon (8) dans le tube d'insertion (2),
et dans lequel, dans la zone de ladite ouverture d'entrée (220), un guide (25) est prévu dans le tube d'insertion (2), de sorte que ledit guide (25) soit ajusté pour guider le piston (3) dans sa direction longitudinale et lors de l'éjection du tampon (8) également dans la direction longitudinale dudit tube d'insertion (2), et dans lequel ledit piston (3), qui est constitué, tout comme ledit tube d'insertion (2), d'un matériau thermoplastique physiologiquement acceptable et suffisamment flexible, c'est-à-dire pliable, qui est adapté à une utilisation répétée, comprend une tige de piston (30), qui est insérée à travers ledit guide (25) du tube d'insertion (2) et est mobile d'avant en arrière le long dudit guide (25), dans lequel ladite tige de piston (30) est, sur sa partie terminale (301) tournée vers le tube d'insertion (2), munie d'une section de poussée (31) épaissie et de forme appropriée, adaptée pour reposer sur la surface du tampon (8) à l'intérieur du tube d'insertion (2), tandis que sur sa partie terminale restante (32), qui est située à l'extérieur du tube d'insertion (2), la tige de piston (30) est équipée d'une section de commande (33), qui est ergonomiquement ajustée pour être en contact avec un doigt humain afin de permettre la commande du mouvement du piston (3) le long du tube d'insertion (2) lors de la poussée du tampon (8) hors du tube d'insertion (2) dans une cavité corporelle, ou également pour pousser simplement le piston (3) lui-même vers l'intérieur du tube d'insertion (2), dans lequel la longueur de ladite tige de piston (30) est déterminée de telle sorte qu'en respectant tous les paramètres ergonomiques nécessaires à la manipulation avec l'applicateur (1), le piston (3) puisse encore être déplacé au moins sur une distance suffisante pour éjecter le tampon (8) hors du tube d'insertion (2),
et dans lequel ladite cartouche de protection (4), qui est constituée, tout comme le tube d'insertion (2) et le piston (3), d'un matériau thermoplastique physiologiquement acceptable, flexible et pliable, qui est également adapté à une utilisation répétée, est conçue comme un boîtier creux fermé sur toutes ses faces, mais est pourvu d'une ouverture d'entrée (40), à travers laquelle peut être insérée, de manière étanche et protégée contre un retrait non souhaité, au moins la partie du tube d'insertion (2) de l'applicateur (1) qui, lors de l'utilisation, c'est-à-dire juste avant l'éjection du tampon (8) stocké à l'intérieur du tube d'insertion (2), doit être insérée dans une cavité corporelle,
dans lequel le tube d'insertion (2) est, dans la zone autour de ladite ouverture d'entrée (22), sur sa partie d'extrémité arrière (22) et à une distance suffisante de l'ouverture de sortie (210) sur sa partie terminale d'insertion (21), qui est supérieure à la longueur du tampon (8), pourvu d'une bride rigide (23),
et dans lequel, dans la zone de ladite bride (23), le guide (25) de la tige de piston (30) du piston (3) est entouré d'un boîtier (250) suffisamment rigide et flexible, qui est immobilisé de manière amovible à ladite bride (23) du tube d'insertion (2), dans une position dans laquelle la tige de piston (30) du piston (3) et le tube d'insertion (2) sont agencés de manière coaxiale l'un par rapport à l'autre, et ce de sorte que le guide (25) soit protégé contre tout retrait non souhaité du tube d'insertion (2), que ce soit lors de la poussée du piston (3) pour l'éjection du tampon (8) hors du tube d'insertion (2) pendant son insertion dans une cavité corporelle, ou également lors du retrait du tube d'insertion (2) lui-même d'une cavité corporelle,
et dans lequel ledit boîtier (250) du guide (25) peut être fixé à la bride (23) du tube d'insertion (2) et y est immobilisé au moyen d'une coopération mutuelle d'une part entre une rainure (231) s'étendant de manière circonférentielle qui est présente loin de la partie terminale d'insertion (21) du tube d'insertion (2) faisant face à la surface externe (230) de la bride (23) et d'autre part une nervure (252) s'étendant de manière circonférentielle qui est présente sur la surface interne (251) du boîtier (250) du guide (25) du piston (3), qui doit être immobilisée dans ladite rainure (231),
et dans lequel ladite nervure (252) peut, en variante, être agencée sur la bride (23), tandis que ladite rainure (231) est agencée sur le boîtier (250) du guide (25),
**caractérisé en ce que**
la bride (23) du tube d'insertion (2) est fixée en pivotement au boîtier (250) du guide (25), de sorte que le guide (25), conjointement avec la tige de piston (30) du piston (3), qui traverse ledit guide (25), puisse se déplacer entre sa première position terminale, dans laquelle le tube d'insertion (2) et la tige de piston (30) sont alignés de manière coaxiale, et sa deuxième position terminale, dans laquelle les axes géométriques longitudinaux du tube d'insertion (2) et de la tige de piston (30) s'étendent parallèlement l'un à l'autre, ou dans toute autre position intermédiaire entre lesdites positions terminales si souhaité.

2. Applicateur selon la revendication 1, **caractérisé en ce que** ladite bride (23) du tube d'insertion (2) et ledit boîtier (250) du guide (25) de la tige de piston (30) du piston (3) sont reliés solidement et de manière permanente l'un à l'autre au moyen d'une liaison flexible (255), qui représente une partie intégrante à la fois du boîtier (250) du guide (25) et simultanément également de la bride (23) du tube d'insertion (2).

3. Applicateur selon la revendication 1, **caractérisé en ce que** ladite bride (23) du tube d'insertion (2) et ledit boîtier (250) du guide (25) du piston (3) sont reliés l'un à l'autre en pivotement, mais de façon détachable.

4. Applicateur selon l'une des revendications 1 à 3, **caractérisé en ce que** la bride (23) est pourvue d'une découpe (235) qui est ajustée pour permettre le passage de la ficelle de retenue (80) à travers celle-ci, lorsque le tampon (8) est chargé dans le tube d'insertion (2), vers l'extérieur dudit tube d'insertion (2) de l'applicateur (1).

5. Applicateur selon l'une des revendications 1 à 4, **caractérisé en ce que**, dans une position où le piston (3) est entièrement poussé vers l'intérieur du tube d'insertion (2) jusqu'à une position terminale de celui-ci, la tige de piston (30) est immobilisée et sécurisée contre tout mouvement incontrôlé dans la direction longitudinale de l'applicateur (1).

6. Applicateur selon la revendication 5, **caractérisé en ce que**, aux fins de ladite protection contre tout mouvement incontrôlé de la tige de piston (30) dans la partie terminale du piston (3), lorsque celui-ci est complètement poussé vers l'intérieur du tube d'insertion (2), une rainure (258) s'étendant de manière circonférentielle est agencée sur la surface interne (257) du guide (25), et une nervure (311) s'étendant de manière circonférentielle est agencée sur la surface externe (310) de la tige de piston (30) à proximité immédiate de la section de commande (33), qui peut être immobilisée dans ladite rainure (258) sur le guide (25).

7. Applicateur selon la revendication 5, **caractérisé en ce que,** en raison de la protection de la tige de piston (30) contre tout mouvement non souhaité de celle-ci dans ladite position terminale sur la surface interne (257) du guide (25), une rainure (258) est prévue sur la tige de piston (30) tandis que, sur le guide (25), une nervure (311) s'étendant de manière circonférentielle est présente, qui est ajustée pour coopérer avec ladite rainure (258).

8. Applicateur selon l'une quelconque des revendications 1 à 7, **caractérisé en ce que** le tube d'insertion (2) peut être inséré de manière étanche dans la cartouche de protection (4) et est, dans son état inséré, bloqué et protégé contre tout retrait non souhaité grâce à sa surface externe conique (200).

9. Applicateur selon l'une quelconque des revendications 1 à 7, **caractérisé en ce que** le tube d'insertion (2) peut être inséré de manière étanche dans la cartouche de protection (4) et est, dans son état inséré, bloqué et protégé contre tout retrait non souhaité au moyen d'au moins une rainure (401) s'étendant de manière circonférentielle sur la surface interne (400) de la cartouche de protection (4), qui est adaptée pour coopérer avec au moins une nervure (201) s'étendant de manière circonférentielle sur la surface externe (200) du tube d'insertion (2).

10. Applicateur selon l'une quelconque des revendications 1 à 9, **caractérisé en ce que** le tube d'insertion (2), pour recevoir le tampon (8), et le piston (3), pour pousser le tampon (8) à travers l'ouverture d'éjection (210) du tube d'insertion (2), sont ajustés pour coopérer avec un tampon (8) qui est soit un tampon avec applicateur, soit un tampon sans applicateur.
